# EUROPEAN PATENT APPLICATION

(11) **EP 2 674 109 A1**
(43) Date of publication of application: **18.12.2013**
(21) Application number: 12172243.3
(22) Date of filing: 15.06.2012
(51) Int. Cl.: A61B 17/06

(54) **Endoscopic surgical apparatus**

(71) Applicant: Endo Tools Therapeutics S.A., 1400 Nivelles (BE)
(72) Inventor: Hiernaux, Martin, 1170 Watermael-Boitsfort (BE); Chau, Alexandre, 1081 Koekelberg (BE); Huberty, Vincent, 1180 Uccle (BE); Ibrahim, Mostafa, 12311 Giza (EG); Deviere, Jacques, 7090 Braine Le Comte (BE)
(74) Representative: De Groote, Christophe

(57) **Abstract**

Endoscopic surgical apparatus comprising an anchoring means (5) for anchoring a living tissue, and a piercing means (3) for piercing said living tissue, each means being driven by an independent driving means. The anchoring means (5) and the piercing means (3) are able to move along parallel axes that extend outside a distal end (1) of a catheter, allowing piercing a living tissue on a short length without having to fold the living tissue.

## Description

### Field of the invention

The invention relates an endoscopic surgical apparatus comprising:
- a hollow tube with a distal end and a proximal end,
- a piercing means at the distal end for piercing a tissue wall,
- a first driving means for moving the piercing means back and forward along a piercing axis,
- an anchor means at the distal end for anchoring the tissue wall,
- a second driving means, for moving the anchor means back and forward along an anchoring axis,
- the first driving means being different from the second driving means.

### Description of prior art

Such endoscopic apparatus (sometimes also called "endoscope") are well known in the art and are used to perform minimally invasive surgical acts. As the diameter of the endoscope is limited to the size of the natural orifices in which the endoscope needs to be introduced, the number of surgical devices at the distal end of the endoscope is limited. Endoscopic procedures include the suture of an internal tissue wall of interest. Such suturing comprises the passage of suture material in and out of the tissue wall at least once, but more often several times. The suturing steps may involve a difficult manipulation of the device, and can be a challenging task. Furthermore, it is essential that the piercing occurs on very located spots of the tissue wall for an optimal suturing. Because internal tissues are soft matters, it can be very challenging to effectively pierce the tissue wall on such located spots. Positioning precisely the piercing means relative to the tissue wall may be very time consuming and the tissue wall may move during the piercing step. A solution may be to add an anchor means at the distal end of the endoscopic surgical apparatus. Anchoring the tissue wall before and while piercing the tissue wall permits to facilitate a piercing step needed to complete a suture along with a better accuracy of piercing.

US20050251157 discloses for example an endoscopic apparatus in which an anchor and grabbing means is present at the distal end of the endoscopic apparatus. The anchor and grabbing means has the shape of a corkscrew which extends distally from the distal end of the endoscope. In operation, the anchor means first grasps a tissue wall, and then folds it in order to make a plication. Then, the piercing means - here a needle - penetrates the tissue walls. In order to pierce the shortest hole in the tissue wall, the tissue wall has to be strongly folded. This may be very harmful for the tissue wall. Moreover, in this configuration, the piercing means pierce the tissue wall twice. If the tissue wall has to be pierced only one time, the piercing means will have to pierce the tissue wall slanted, so that the length of the piercing hole in the tissue wall will be longer than the length of a piercing hole that is substantially perpendicular to the tissue wall, which is more harmful for the tissue wall and which also increases the difficulty of the piercing itself. Furthermore, if the tissue wall is thick, this apparatus may be unable to correctly fold the tissue wall, which may lead to inaccuracy of piercing. Such endoscopic apparatus may not be able to fold a thick tissue wall due to the presence of an upper bail and a lower bail. With this configuration, a thick tissue wall can not be pierced longitudinally.

There is also a need for a device that may pierce a tissue wall in a secured manner without folding and/or bending the tissue wall with an apparatus linked to the piercing apparatus. When both apparatus are linked, the localization of the piercing spot is dependent on the distance between the plication means and the piercing means, and the piercing spot is hence not freely selected after the plication has been performed.

### Summary of the invention

It is an object of the invention to provide an endoscopic surgical apparatus that resolves, at least in part, the limitations of the prior art. It is an object of the invention to permit a single substantially perpendicular piercing of a tissue wall with a single apparatus. It is also an object of the invention to limit the risk of collision between the piercing means and the anchoring means.

To this end, the endoscopic surgical apparatus according to the invention is **characterised in that** the piercing axis and the anchoring axis are parallel to each other. With such apparatus, the tissue wall may be pierced transversally without having to fold it because the anchoring axis and the piercing axis are parallel. Such configuration allows having a shorter piercing hole in the tissue wall, reducing the damages to the tissue wall. With such apparatus, the plication of the tissue wall is not required to pierce substantially transversally the tissue wall.

Preferably, the anchoring means has a shape and a size which remain substantially constant over time. This permits to know exactly the relative position of the piercing spot with respect to the anchoring axis. This allows knowing exactly the distance range between the anchoring spot and the piercing spot, and hence the range of cantilever between both.

Preferably, the piercing axis and the anchoring axis are the same axis. This allows reducing the range of the cantilever between the piercing means and the anchoring means when they have respectively the same size and the same shape as a piercing means and an anchoring means that move along parallel but distinct axis. Furthermore, such configuration enables to reduce the section of the endoscopic surgical apparatus, which is advantageous in the field of endoscopic surgery apparatus.

It is a further object of the invention to provide an endoscopic surgical apparatus adapted to capture a suture. To this end, the endoscopic surgical apparatus according to the invention is preferably **characterised in that** the piercing means comprises a hollow needle and a suture grasping means, said grasping means being connected to a third driving means, wherein the hollow needle and the grasping means are adapted to permit independent movement.

### Short description of the drawings

These and further aspects of the invention will be explained in greater detail by way of example and with reference to the accompanying drawings in which:

Fig.1 shows a perspective view of the distal end of an endoscopic surgical apparatus according to the invention.

Fig.2a shows a perspective view of the distal end of the apparatus of Fig.1 before the anchoring step.

Fig.2b shows a perspective view of the distal end of the apparatus of Fig.1 while anchoring the tissue wall.

Fig.2c shows a perspective view of the distal end of the apparatus of Fig.1 while piercing the tissue wall.

Fig.3a shows a perspective view of a preferred embodiment of an apparatus according to the invention.

Fig.3b shows a perspective view of the distal end of the apparatus of Fig.3a before the anchoring step.

Fig.3c shows a perspective view of the distal end of the apparatus of Fig.3a while anchoring the tissue wall.

Fig.3d shows a perspective view of the distal end of the apparatus of Fig.3a while piercing the tissue wall.

Fig.4 shows a perspective view of another preferred embodiment of an apparatus according to the invention.

Fig.5a shows a view of the distal end of an exemplary piercing tip of the anchor means.

Fig.5b shows a view of the distal end of another exemplary piercing tip of the anchor means.

Fig.6a shows a perspective view of the distal end of an endoscopic surgical apparatus according to an alternative embodiment.

Fig.6b shows a perspective view of the distal end of an endoscopic surgical apparatus according to another alternative embodiment.

Fig.6c is a top view of the distal end of the apparatus of Fig.6a.

Fig.6d is a top view of the distal end of the apparatus of Fig.6b.

The figures are not drawn to scale. Generally, identical components are denoted by the same reference numerals in the figures.

### Detailed description of preferred embodiments

The term "lumen" refers to the opening or the inside space of a hollow tubular element facilitating the insertion of a second element or the inside space of an organ or the inside space of a body cavity. The term "lumen" may also refer to the inside space of an organ, such as the inside of a stomach for example.

The term "tissue wall" refers to an organ wall of a living being, such as a stomach wall or a small intestine wall.

The term "distal" refers to the farthest end of an element from the point of origin of the endoscopic apparatus, said point of origin being generally where the command means are disposed.

The term "proximal" refers the closest end of an element from the point of origin of the endoscopic apparatus, said point of origin being generally where the command means are disposed.

A helix is a three-dimensional curve, the tangent to any point of which makes a constant angle with a fixed line generally called the axis of the helix. A circular helix is formed by winding a line around a cylinder so that the radius is constant. A conical helix is formed by winding a line around a cone, so that, consequently, its radius constantly changes.

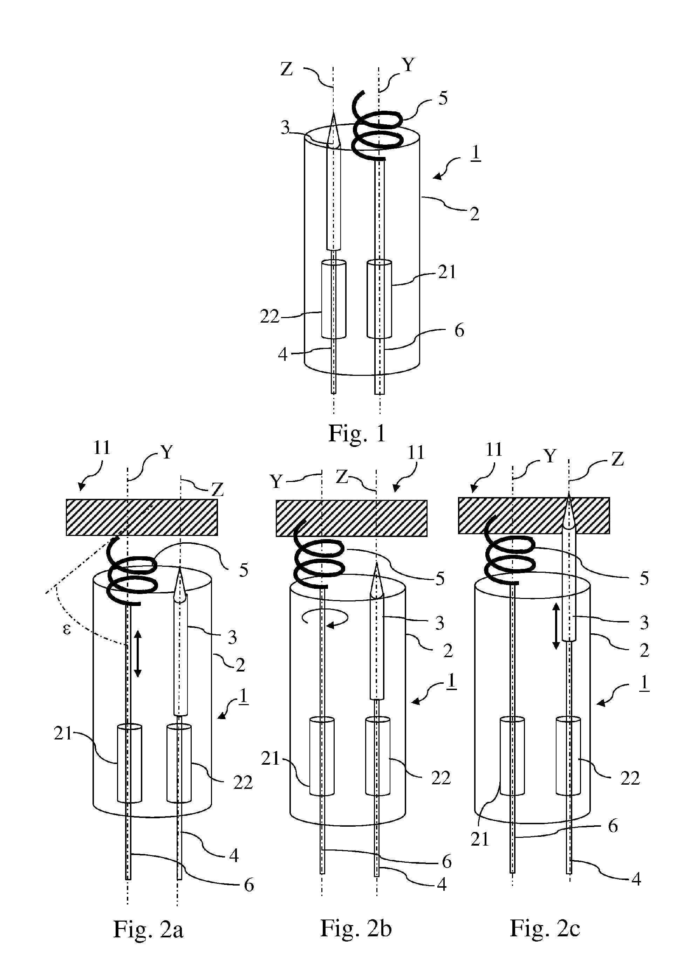

Fig.1 shows a perspective view of the distal end of an endoscopic surgical apparatus according to the invention. As shown on this figure, the distal end of the endoscopic surgical apparatus (1) comprises:
- a hollow tube (2) with a distal end and a proximal end,
- a piercing means (3) for piercing a tissue wall, said piercing means (3) being at the distal end,
- a first driving means (4) for moving the piercing means (3) back and forward along a piercing axis (Z),
- an anchor means (5) for anchoring the tissue wall, said anchoring means (5) being at the distal end,
- a second driving means (6), for moving the anchor means (5) back and forward along an anchoring axis (Y),
- the first driving means (4) being different from the second driving means (6).

The hollow tube (2) may be any kind of tubular body, such as a catheter for example. The hollow tube (2) may be tubular such that at least one lumen is defined through its length. The hollow tube (2) may be flexible or rigid. The distal end of the hollow tube (2) may be fabricated from a flexible material to permit bending of its distal end.

The piercing means (3) may for example be a needle. Preferably, the needle (3) is at least partially hollow such that a suture or a suture grasping device may be positioned inside it for deployment within or upon the tissue wall. A distal tip of the needle may be tapered or sharpened for piercing more easily into the tissue.

The first driving means (4) may for example be a first rod which runs through the length of the hollow tube (2) and to which the piercing means (3) is attached at one end. Manipulation of the first rod (4) permits to move the piercing means (3) along the piercing axis (Z), allowing the piercing means (3) to move forward outside the hollow tube (2) through a tissue wall, or to move backward inside the hollow tube (2).

The second driving means (6) may for example be a second rod which runs parallel with the first rod through the length of the hollow tube (2) and to which the anchor means (5) is attached at one end. Manipulation of the second rod (6) permits to move the anchor means (3) along the anchoring axis (Y), allowing the anchor means (5) to move forward outside the hollow tube (2) up to the tissue wall and then to anchor the tissue wall, or to move backward inside the hollow tube (2).

The anchor means (5) may be adapted to be implanted into or upon a tissue. The anchor means (5) may have different shapes, like a wire round around an axis or a pair of articulated jaws for example.

The first driving means (4) is different from the second driving means (6), allowing independent manipulation of the anchor means (5) and the piercing means (3). With such configuration, the anchor means (5) may move back and forward without any movement of the piercing means (3). With such configuration, the piercing means (3) may move back and forward without any movement of the anchor means (5). It is also possible to move simultaneously the piercing means (3) and the anchor means (5) by manipulation of both the first driving means (4) and the second driving means (6).

The anchoring axis (Y) may be defined as the axis along which the anchor means is movable in translation. Moreover, the anchoring axis may also be the geometric axis of the anchor means. For example, for a helix-shaped anchor means, this axis may be the axis around which the curve circles. A helix may be described as a curve turning about an axis, which is generally the anchoring axis, on the surface of a cylinder or cone while rising at an upward angle from a base of the cylinder. When the anchoring means is a pair of articulated grasping jaws, this anchoring axis may be a straight line that crosses both the articulation of the pair of grasping jaws and the distal ends of both jaws when the jaws are in a closed position.

The piercing axis (Z) may be defined as the axis along which the piercing means is movable in translation. The piercing means and the anchor means may extend substantially distally from the distal end of the hollow tube.

The endoscopic surgical apparatus is **characterized in that** the piercing axis (Z) and the anchoring axis (Y) are parallel to each other. For example, as shown in **fig.1****,** the piercing means (3) is mounted at a distal end of the first rod (4), in the continuation/lengthening of said first rod (4), and said first rod (4) is snugly fitted in a first sheath (22). Hence, by pushing or pulling the first rod, the piercing means (3) will move in translation along the piercing axis (Z). The anchoring means (5) is mounted at an end of the second rod (6), in the continuation/lengthening of said second rod (6), and said second rod (6) is snugly fitted in a second sheath (21). Hence, by pushing or pulling the second rod, the anchoring means (5) will move in translation along the anchoring axis (Y). In this example, the first sheath (22) and the second sheath (21) are hollow cylinders which are fit parallel to each other inside the hollow tube (2) at its distal end, allowing the movement according to parallel axes of the piercing means (3) and the anchoring means (5).

Preferably, the anchoring means has at rest a constant shape and a constant size. For example, the anchoring means is in a metallic material like stainless steel, titanium or nitinol (nickel titanium). For example, the anchoring means is not in a shape-memory material or a shape-memory alloy that changes shape when being warmed by the body heat. This permits to know exactly the range of positions of the piercing spot with respect to the anchoring spot. This allows knowing more precisely at which depth the distal end of the piercing means is in the tissue wall when anchoring. Moreover, this allows securing the piercing spot before piercing occurs. Moreover, this permits to know exactly the maximum distance and the minimum distance between the anchoring spot and the piercing spot.

**Fig.2a, fig.2b** and **fig.2c** show how the apparatus of fig.1 can be operated to anchor and pierce a tissue wall. The hollow tube (2) may be advanced into a body lumen up to near a tissue wall (11) as shown in **fig.2a****.** When the desired spot to be pierced has been reached and the distal end of the hollow tube (2) has been desirably positioned relative to the tissue wall (11), the anchor means (5) may be moved forward by manipulating the second driving means (6) such that the anchor means (5) penetrates the tissue wall (11) at a desired spot. The manipulation of the second driving means (6) permits to move the anchor means (5) in translation along the anchoring axis (Y). Once the tissue wall (11) is reached, rotation of the driving means (6) permits to rotate the anchoring means (5) for anchoring it into the tissue wall (11). Once the distal end of the anchor means (5) has been anchored into the tissue wall **(****fig.2b****),** a manipulation of the first driving means (4) permits to move the piercing means (3) in translation along the piercing axis (Z). Once the piercing means (3) reaches the tissue wall (11), it is made to penetrate the tissue wall (11) by manipulation of the first driving means (4) **(****fig.2c****).**

Because a differential force may be acted between the piercing means (3) and the anchor means (5) to permit relative movement of each other, the piercing means may pierce the tissue wall easily. Furthermore, the piercing means (3) and the anchor means (5) being positioned on the distal end of a sole hollow tube (2) and the piercing means (3) and the anchor means (5) moving along parallel axis (respectively Z and Y), the piercing occurs transversally in the tissue wall.

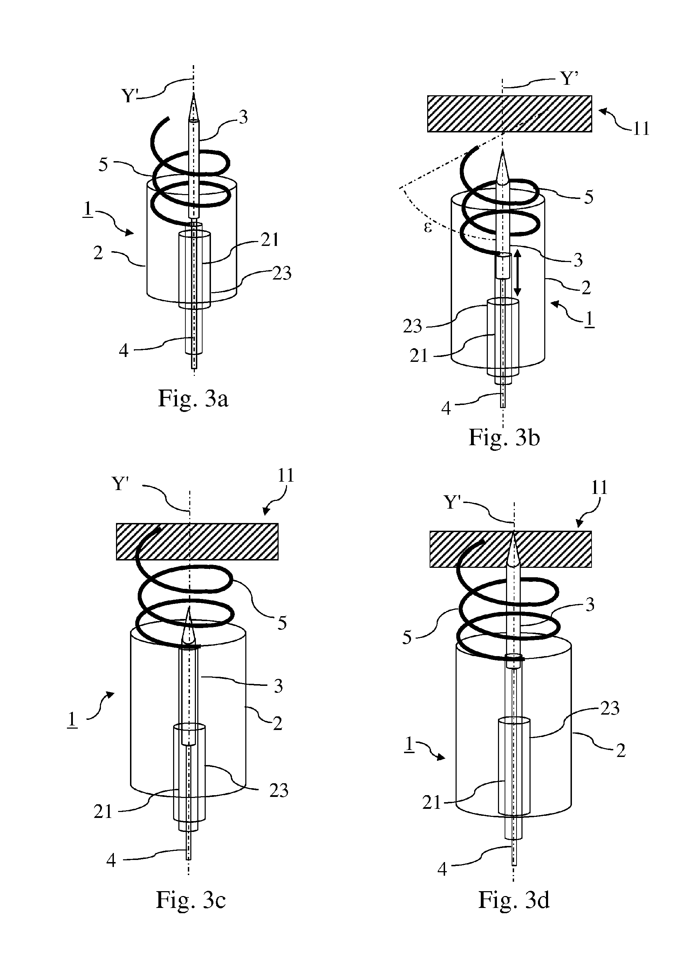

**Fig.3a** shows a perspective view of a preferred embodiment of an apparatus according to the invention. Here, the piercing means (3) is also mounted at a distal end of the first rod (4), in the continuation/lengthening of said first rod (4), and said first rod (4) or said piercing means (3) is snugly fitted in a first sheath (21). The anchoring means (5) is mounted at a distal end and in the continuation/lengthening of said first sheath (21), and said first sheath (21) snugly fits into a second sheath (23). Preferably, the first rod (4), the first sheath (21), and the second sheath (23) are coaxial cylinders. Preferably, the piercing means (3) and the anchoring means (5) are coaxial with the first rod (4), the first sheath (21), and the second sheath (23). Accordingly, the piercing means and the anchoring means may be operated independently of each other by respectively manipulating the first rod (4) and the first sheath (21), and the piercing axis (Z) and the anchoring axis (Y) will be the same axis (Y'). This permits to know exactly the maximum distance and the minimum distance between the anchoring spot and the piercing spot, allowing a more accurate localisation of the piercing spot relative to the anchoring spot. Furthermore, with this preferred embodiment, the distance between the piercing spot and the anchoring spot may be reduced because the anchoring spot may be closer to the piercing spot.

An example of how the apparatus of fig.3a can be operated to anchor and pierce a tissue wall (11) is seen in **fig.3b, fig.3c** and **fig.3d****,** which respectively correspond to fig. 2a, fig.2b and fig. 2c.

In a preferred embodiment, the anchor means (5) has the shape of a space curve wound round the anchoring axis (Y, Y'). For example, the anchor means may be a gimlet or a corkscrew.

In a more preferred embodiment, as shown in **fig.3a** for example, the anchor means (5) has a helix-shaped member as its distal end whose main axis corresponds to the anchoring axis (Y or Y'). Such embodiment permits to anchor the anchor means (5) at a desired depth into the tissue wall (11).

The helix may be a circular helix, as shown in **fig.3a****.**

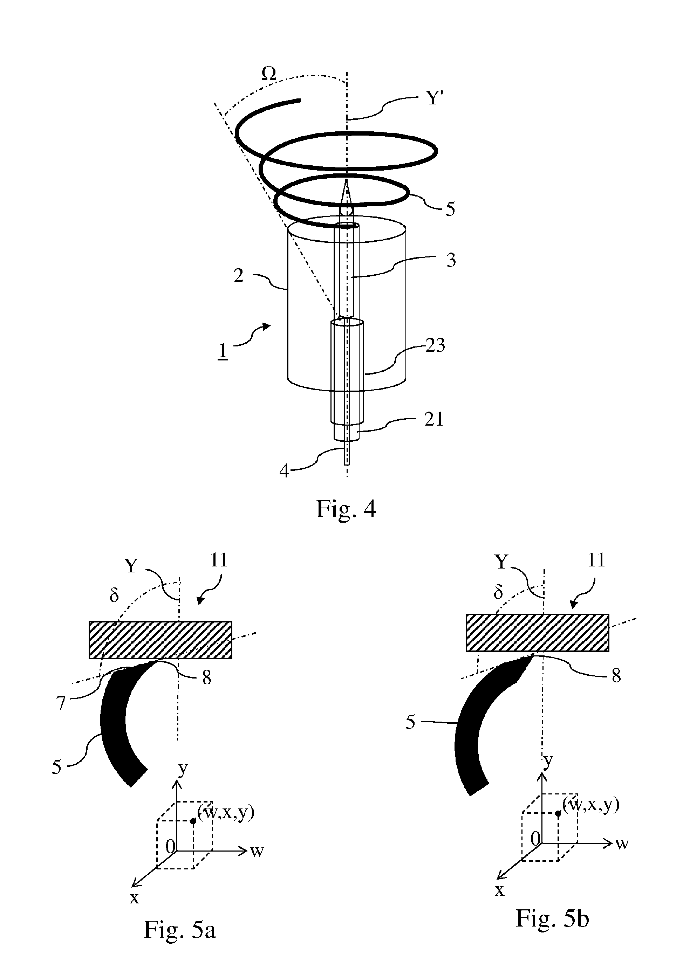

Alternatively, as seen in **fig.4****,** the anchor means (5) may be a conical helix whose base of the cone around which the helix is rounded is at the distal end of the anchor means (5). In a more preferred embodiment, an angle Ω between the anchoring axis (Y, Y') and the slope of the cone around which the conical helix is rounded is comprised between 0° and 45°. Such embodiments permit to reduce the risk of contact between the piercing means (3) and the anchor means (5) in case one of said means (3 or 5) is deflected from its nominal movement axis.

In a more preferred embodiment, a slope of the helix-shaped anchor means (5) is constant. A curve of constant slope is defined by the property that a tangent at any point of the curve makes a constant angle with a fixed straight line (the axis of the helix). Such embodiment permits to reduce the risk of deformation of the tissue wall by the anchor means (5) if said wall is thicker than a pitch of the helix-shaped anchor.

In a preferred embodiment, as shown in **fig.5a** and **fig.5b****,** the distal end the anchor means (5) has a piercing tip (8), in order to easily penetrate the tissue wall (11). Said piercing tip (8) may have the shape of any known piercing tip. For example, said piercing tip may be sharp.
Preferably, as shown in **fig.5b****,** the distal end of the anchor means has a shape of a tip whose end point has the largest y coordinate in an orthogonal 3D referential having the distal end of the hollow tube as origin and the anchoring axis as Y axis, said Y axis being oriented from said distal end towards the anchoring means.

Said piercing tip may also be tapered, bevelled or sharpened in order to penetrate the tissue more easily. As seen in **fig.5a****,** the distal end of the anchor means has a bevelled part (7) to permit the piercing tip to penetrate the tissue wall first. For example, the angle δ, which is the angle between the anchoring axis and the bevelled part (7) of the piercing tip, is superior to 90° and inferior to 180°. In a more preferred embodiment, δ is comprised between 90° (not included) and 135° (included).

If the distal end of the anchor means is not bevelled, as shown in fig.5b, the angle δ, which is the angle between the anchoring axis (Y, Y') and a longitudinal side of the piercing tip (8), is preferably superior to 90° and inferior to 180°. In a more preferred embodiment, δ is comprised between 90° (not included) and 135° (included).

In a preferred embodiment, as shown in **fig.3b****,** the angle ε between a tangent line at a point of the helix and the anchoring axis of the helix-shaped anchor, is superior or equal to 45° and inferior to 90°. With such embodiment, the distal end of the anchor means is capable of penetrating the tissue wall in a slanted manner.

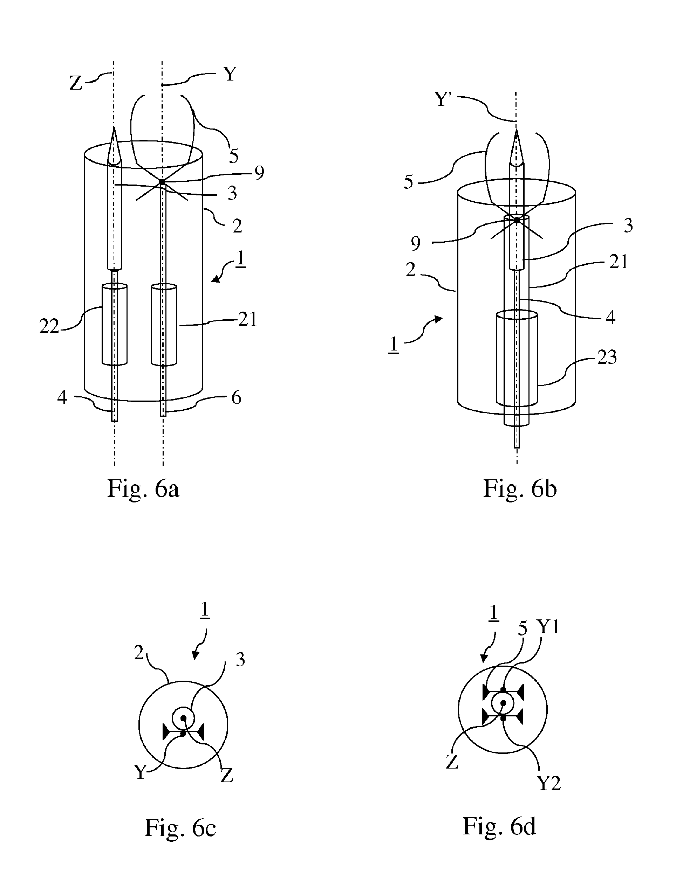

In an alternative embodiment, as shown in **fig.6a** and **6c****,** the anchor means (5) comprises a pair of articulated grasping jaws. Any kind of grasping jaws may be used like alligators jaws, prong shape jaws, pelican shape jaws or triangular jaws. The number of grasping jaws is not limited to one pair. The anchor means may comprise two pairs of grasping jaws, as shown in **figs.6b** and **6d** for example. The articulation (9) of the pair of grasping jaws and the distal ends of the grasping jaws when the jaws are closed are preferably in the prolongation of the distal end of the second driving means (6) to define the anchoring axis (Y, Y1, Y2).

When the anchor means are a pair of grasping jaws, each grasping jaw may be located on either longitudinal side of the piercing means.

In an alternative embodiment, the pair of grasping jaws may contain a partially hollow space when the jaws are closed in order to hold in the piercing means in a hollow space when the jaws are closed.

In a preferred embodiment of the invention, the endoscopic surgical apparatus is adapted to capture a suture. To this end, the endoscopic surgical apparatus according to the invention is preferably **characterised in that** the piercing means comprises a hollow needle and a suture grasping means, said grasping means being connected to a third driving means, wherein the hollow needle and the grasping means are adapted to permit independent movement. The needle is preferably a hollow tubular needle through which one or several suture grasping means may be delivered. A suture grasping means may be connected to a third driving means, wherein the hollow needle and the grasping means are driven to permit relative movement therebetween. The third driving means may be a control wire or a rod or a combination of a control wire and a rod which run through the length of the hollow tube (2) and having the suture grasping means at one end. A rod may be present between the control wires and the suture grasping means. Manipulation of the third driving means permits to move the suture grasping means, allowing the suture grasping means to move forward outside the hollow needle or to move backward inside the hollow needle. The first driving means and the third driving means are different, allowing independent manipulation of the piercing means and the suture wire grasping means. With such configuration, the piercing means may move back and forward without any movement of the suture wire grasping means. With such configuration, the suture wire grasping means may move back and forward without any movement of the piercing means. It is also possible to move simultaneously the piercing means and the suture wire grasping means by manipulation of both driving means. A grasping means may be a spring-loaded hoop member or a loop that may be selectively deployed at least partially out of a hollow piercing means. The spring-loaded member or the loop may be selectively retracted at least partially inside of a hollow needle.

The present invention has been described in terms of specific embodiments, which are illustrative of the invention and not to be construed as limiting. More generally, it will be appreciated by persons skilled in the art that the present invention is not limited by what has been particularly shown and/or described hereinabove. The invention resides in each and every novel characteristic feature and each and every combination of characteristic features.
Reference numerals in the claims do not limit their protective scope.
Use of the verbs "to comprise", "to include", "to be composed of", or any other variant, as well as their respective conjugations, does not exclude the presence of elements other than those stated.
Use of the article "a", "an" or "the" preceding an element does not exclude the presence of a plurality of such elements.

Summarized, the invention may also be described as follows:
an endoscopic surgical apparatus comprising an anchoring means for anchoring a living tissue, and a piercing means for piercing said living tissue,
each means being driven by an independent driving means. The anchoring means and the piercing means are able to move along parallel axes that extend outside a distal end of a catheter, allowing piercing a living tissue on a short length without having to fold the living tissue.

## Claims

1. An endoscopic surgical apparatus comprising
- a hollow tube (2) with a distal end (1) and a proximal end,
- a piercing means (3) at the distal end (1) for piercing a tissue wall (11),
- a first driving means (4) for moving the piercing means (3) back and forward along a piercing axis (Z),
- an anchor means (5) at the distal end (1) for anchoring the tissue wall,
- a second driving means (6), for moving the anchor means (5) back and forward along an anchoring axis (Y),
- the first driving means (4) being different from the second driving means (6),
**characterized in that**
the piercing axis (Z) and the anchoring axis (Y) are parallel to each other.

2. An endoscopic surgical apparatus according to claim 1, **characterized in that** the anchor means (5) has a shape of a space curve winded round the anchoring axis (Y).

3. An endoscopic surgical apparatus according to claim 1 or 2, **characterized in that** a distal end of the anchor means (5) has the shape of a helix whose axis corresponds to the anchoring axis (Y).

4. An endoscopic surgical apparatus according to claim 3, **characterized in that** the helix is a circular helix.

5. An endoscopic surgical apparatus according to claim 3, **characterized in that** the helix is a conical helix whose base of the cone around which said conical helix is rounded is at the distal end of the anchor means (5).

6. An endoscopic surgical apparatus according to anyone of the previous claims, **characterized in that** the piercing axis (Z) and the anchoring axis (Y) are the same axis.

7. An endoscopic surgical apparatus according to anyone of the previous claims, **characterized in that** the distal end of the anchor means (5) has a shape of a tip whose end point has the largest y coordinate in an orthogonal 3D referential having the distal end (1) of the hollow tube (2) as origin and the anchoring axis as Y axis, said Y axis being oriented from said distal end towards the anchoring means (5).

8. An endoscopic surgical apparatus according to anyone of the claims 3-5;7;
and 6 when it depends on claims 3 to 5, **characterized in that** the angle between a tangent line at a point of the helix and the axis of the helix is superior or equal to 45° and inferior to 90°.

9. An endoscopic surgical apparatus according to claim 1, **characterized in that** the anchor means (5) comprises a pair of articulated grasping jaws.

10. An endoscopic surgical apparatus according to claim 1, **characterized in that** the anchor means (5) comprises at least two pairs of articulated grasping jaws.

11. An endoscopic surgical apparatus according to anyone of the previous claims, **characterized in that** the piercing means (3) comprises a hollow needle.

12. An endoscopic surgical apparatus according to claim 11, **characterized in that** the piercing means (3) comprises a hollow needle and a suture grasping means, said grasping means being connected to a third driving means, wherein the hollow needle and the grasping means are adapted to permit independent movement.
